# EUROPEAN PATENT SPECIFICATION

(11) **EP 4 255 543 B1**
(45) Date of publication and mention of the grant of the patent: **21.01.2026**
(21) Application number: 21899331.9
(22) Date of filing: 28.10.2021
(51) Int. Cl.: A61M 16/10, A61M 16/00, A61M 16/06, B01D 46/00

(54) **AIR FILTRATION APPARATUS AND SYSTEM FOR PROVIDING PATIENT INFORMATION**
LUFTFILTERVORRICHTUNG UND SYSTEM ZUR BEREITSTELLUNG VON PATIENTENINFORMATIONEN
APPAREIL DE FILTRATION D'AIR ET SYSTÈME DE FOURNITURE D'INFORMATIONS DE PATIENT

(30) Priority: 04.12.2020 AU 2020904496
(43) Date of publication of application: 11.10.2023
(73) Proprietor: ResMed Pty Ltd, Bella Vista, New South Wales 2153 (AU)
(72) Inventor: CORK, Simon Robert, Bella Vista, New South Wales 2153 (AU); RICHARDS, David Andrew, Bella Vista, New South Wales 2153 (AU)
(74) Representative: Vossius & Partner Patentanwälte Rechtsanwälte mbB
(86) International application number: PCT/AU2021/051252
(87) International publication number: WO 2022/115899

(56) References cited:
- WO-A1-2011/050626
- WO-A1-2011/050626
- WO-A1-2017/103246
- WO-A2-2017/032873
- CN-A- 102 389 611
- CN-A- 103 948 998
- FR-A3- 3 067 251
- US-A1- 2014 373 846
- US-A1- 2018 001 049
- US-B2- 8 469 028
- US-B2- 8 469 028
- US-B2- 8 622 057

## Description

### 1 BACKGROUND OF THE TECHNOLOGY

### 1.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 1.2 DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient CO₂ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

Patients in need of respiratory therapy are often sensitive to air quality. Supplying air which contains pollution and/or other particles to such a patient may reduce the effectiveness of the therapy and/or may cause or exacerbate other problems with the patient's respiratory system.

### 1.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 1.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 1.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired CO₂ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched gas at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 1.2.2.3 Supplementary oxygen

For certain patients, oxygen therapy may be combined with a respiratory pressure therapy or HFT by adding supplementary oxygen to the pressurised flow of air. When oxygen is added to respiratory pressure therapy, this is referred to as RPT with supplementary oxygen. When oxygen is added to HFT, the resulting therapy is referred to as HFT with supplementary oxygen.

### 1.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 1.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

### 1.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 cmH₂O).

| RPT Device name | A-weighted sound pressure level dB(A) | Year (approx.) |
|---|---|---|
| C-Series Tango^{™} | 31.9 | 2007 |
| C-Series Tango^{™} with Humidifier | 33.1 | 2007 |
| S8 Escape^{™} II | 30.5 | 2005 |
| S8 Escape^{™} II with H4i^{™} Humidifier | 31.1 | 2005 |
| S9 AutoSet^{™} | 26.5 | 2010 |
| S9 AutoSet^{™} with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar^{™} Series of Adult and Paediatric Ventilators may provide support for invasive and non-invasive non-dependent ventilation for a range of patients for treating a number of conditions such as but not limited to NMD, OHS and COPD.

The ResMed Elisée^{™} 150 ventilator and ResMed VS III^{™} ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

RPT devices may incorporate filters to remove some particles from the air flow supplied to the patient. Such filters are typically only effective at removing relatively large particles. Substitution of the filters of existing RPT devices with more effective filters (i.e. filters capable of filtering smaller particles) may cause an unacceptable increase in impedance to air flow and a consequent reduction in flow rate of the RPT device. To overcome this reduction in flow rate a more powerful blower might be required, which may increase power consumption and noise generation.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 1.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 1.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air. Humidifiers therefore often have the capacity to heat the flow of air was well as humidifying it.

A range of artificial humidification devices and systems are known, however they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore medical humidifiers may have more stringent safety constraints than industrial humidifiers

While a number of medical humidifiers are known, they can suffer from one or more shortcomings. Some medical humidifiers may provide inadequate humidification, some are difficult or inconvenient to use by patients.

### 1.2.3.5 Oxygen source

Experts in this field have recognized that exercise for respiratory failure patients provides long term benefits that slow the progression of the disease, improve quality of life and extend patient longevity. Most stationary forms of exercise like tread mills and stationary bicycles, however, are too strenuous for these patients. As a result, the need for mobility has long been recognized. Until recently, this mobility has been facilitated by the use of small compressed oxygen tanks or cylinders mounted on a cart with dolly wheels. The disadvantage of these tanks is that they contain a finite amount of oxygen and are heavy, weighing about 50 pounds when mounted.

Oxygen concentrators have been in use for about 50 years to supply oxygen for respiratory therapy. Traditional oxygen concentrators have been bulky and heavy making ordinary ambulatory activities with them difficult and impractical. Recently, companies that manufacture large stationary oxygen concentrators began developing portable oxygen concentrators (POCs). The advantage of POCs is that they can produce a theoretically endless supply of oxygen. In order to make these devices small for mobility, the various systems necessary for the production of oxygen enriched gas are condensed. POCs seek to utilize their produced oxygen as efficiently as possible, in order to minimise weight, size, and power consumption. This may be achieved by delivering the oxygen as series of pulses or "boli", each bolus timed to coincide with the start of inspiration. This therapy mode is known as pulsed or demand (oxygen) delivery (POD), in contrast with traditional continuous flow delivery more suited to stationary oxygen concentrators.

### 1.2.3.6 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 1.2.3.7 Vent Technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

### 1.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

US 8 469 028 B2 relates to a respirator assembly composed of a respirator, an air purifier, and a connection tube. The respirator includes a first housing having an inflow unit and an air intake. A first pumping unit is located in the first housing for sucking the air through the inflow unit and exhausting the air through the air outtake. The air purifier includes a second housing having an air intake and an outflow unit. A second pumping unit is located in the second housing for sucking the air through the air intake and exhausting the air through the outflow unit. The outflow unit includes a natural-air outlet and a respirator outlet. The connection tube includes two ends, one of which is connected with the respirator outlet and the other is connected with the inflow unit.

### 2 BRIEF SUMMARY OF THE TECHNOLOGY

The invention is defined by the appended set of claims.

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

Another aspect of certain forms of the present technology relates to apparatus and/or methods of reducing particulates in air supplied to a patient.

One form of the present technology comprises an air filtration apparatus for supplying a flow of filtered air to an inlet of an RPT device, the air filtration apparatus comprising an inlet flow path, an outlet flow path, and a blower configured to receive air from the inlet flow path and to supply air at above ambient pressure to the outlet flow path, wherein the inlet flow path comprises a filter means comprising a HEPA filter and/or a VOC filter, and wherein the blower is configured to supply pressurised air to the outlet flow path at a flow rate of at least 5 L/min.

In examples:
a) the filter means comprises a HEPA filter which is configured to have an efficiency of at least 99% for particles 0.3µm or larger;
b) the apparatus is configured for use with a predetermined RPT device and/or with a device having a predetermined shape around an inlet of the RPT device;
c) a portion of the surface of the apparatus surrounding the outlet is non-planar;
d) the air filtration apparatus comprises one more spacer portions which protrude from a surface portion surrounding the air outlet of the air filtration apparatus;
e) the air filtration apparatus comprises an air quality sensor;
f) the air quality sensor is configured to measure particulate levels and/or VOC levels in the ambient air surrounding the apparatus;
g) the apparatus comprises a further air quality sensor downstream of the filter means;
h) the apparatus comprises a data communication system;
i) the data communication system is a wireless data communication system; and/or
j) the data communication system communicates data related to one or more of: the flow rate through the apparatus; the level of particulates and/or VOCs in the ambient air; and the level of particulates and/or VOCs downstream of the filter means.

Another aspect of one form of the present technology is a system for providing air to a patient at positive pressure for respiratory therapy, wherein the air supplied to the patient has a reduced number of particles of diameter 0.3µm or less compared to the ambient air.

Another aspect of one form of the present technology is a system for providing air to a patient at positive pressure for respiratory therapy, the system comprising an air filtration apparatus comprising a HEPA filter and/or a VOC filter and a first blower and an RPT device comprising a second blower, wherein an air outlet of the air filtration apparatus is located relative to an air inlet of the RPT device such that the air inlet of the RPT device receives filtered air from the air outlet of the air filtration apparatus.

In examples:
a) the air filtration apparatus is configured to define a bypass flow path between a surface of the air filtration apparatus and an adjacent surface of the RPT device, wherein the bypass flow path allows ambient air to flow to the air inlet of the RPT device without passing through the air filtration device;
b) the bypass flow path is configured to allow sufficient bypass flow for the RPT device to provide an outlet air flow at a therapeutic pressure and flow rate;
c) the air filtration apparatus comprises a non-planar surface portion surrounding the air outlet of the air filtration apparatus; and/or
d) the air filtration apparatus comprises one more spacer portions which protrude from a surface portion of the air filtration apparatus adjacent the air outlet.

Another aspect of one form of the present technology is a system for providing air to a patient at positive pressure for respiratory therapy, the system comprising:
a filter means configured to remove particles and/or VOCs from the ambient air;
an RPT device configured to receive air which has been filtered by the filter means and to supply the air at a therapeutic pressure and flow rate to a patient interface;
an air quality sensor in contact with ambient air, the air quality sensor configured to measure at least particulate content of the air; and
data communication means for communicating data related to the particulate content of the ambient air supplied to the patient to a remote receiver.

In examples:
a) the system comprises a second air quality sensor downstream of the filter means;
b) the filter means comprises a HEPA filter;
c) the system comprises an air filtration device which is separate from the RPT device; and/or
d) the air filtration device comprises a first blower and the RPT device comprises a second blower.

Another aspect of one form of the technology comprises a system for providing patient information related to respiratory pressure therapy, the system comprising:
one or more memories configured to store an electronic patient record comprising patient data;
one or more processors in communication with the memory, the one or more processors configured to:
   receive data from an air quality sensor associated with a system for providing air to a patient at positive pressure for respiratory therapy;
   store the data in the patient record; and
   provide a user with access to the patient record.

In examples:
a) the one or more processors are configured to receive data from a second air quality sensor associated with the system for providing air to a patient at positive pressure, wherein one of the air quality sensors is in contact with ambient air and the other of the air quality sensors is in contact with air which has been filtered by the system for providing air to a patient at positive pressure;
b) the one or more processors are configured to store data from both air quality sensors in the patient record; and/or
c) the one or more processors are configured to calculate a difference in readings from the air quality sensors and to store data related to the difference in readings in the patient record.

An aspect of certain forms of the present technology is a medical device that is easy to use, e.g. by a person who does not have medical training, by a person who has limited dexterity, vision or by a person with limited experience in using this type of medical device.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 3 BRIEF DESCRIPTION OF THE DRAWINGS

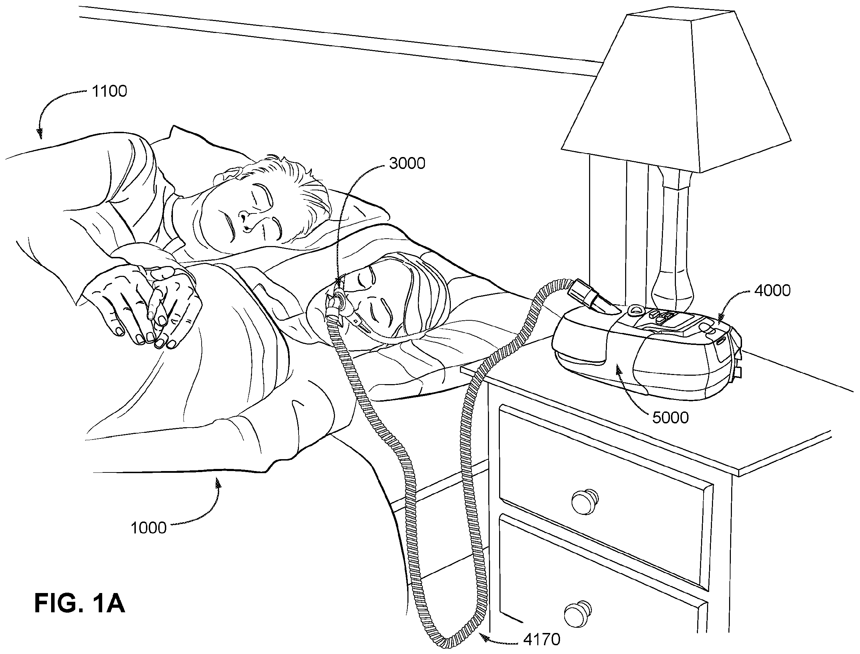

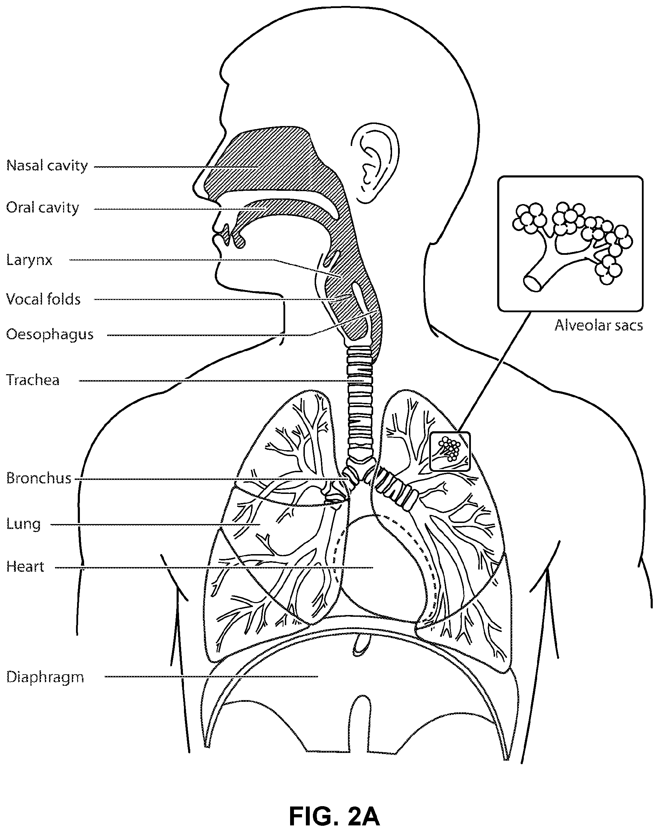

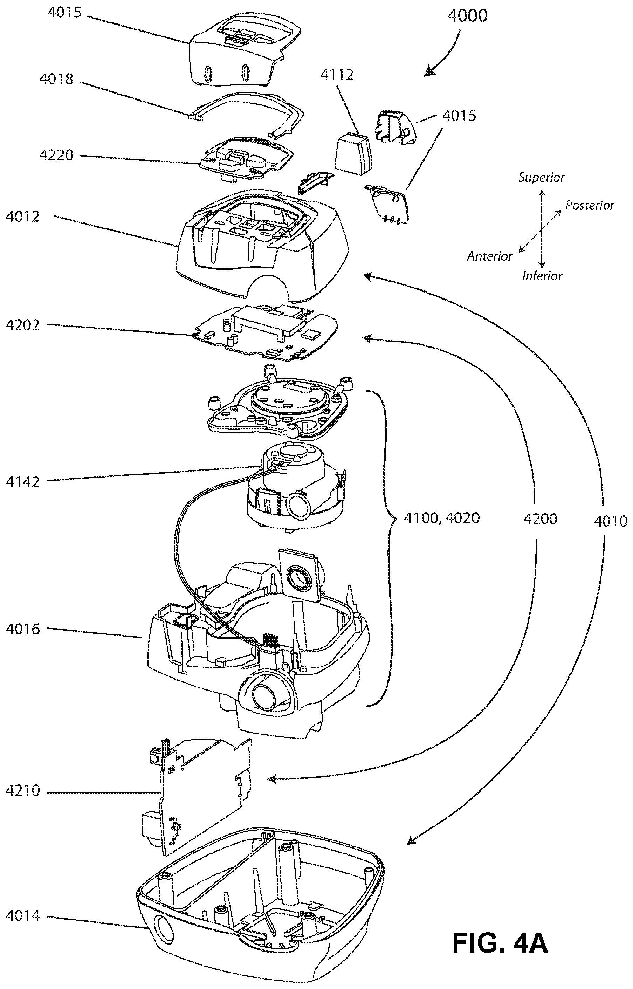

The present technology is illustrated by way of example, and not by way of limitation, in the figures of the accompanying drawings, in which like reference numerals refer to similar elements including:
3.1 RESPIRATORY THERAPY SYSTEMS
   Fig. 1A shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.

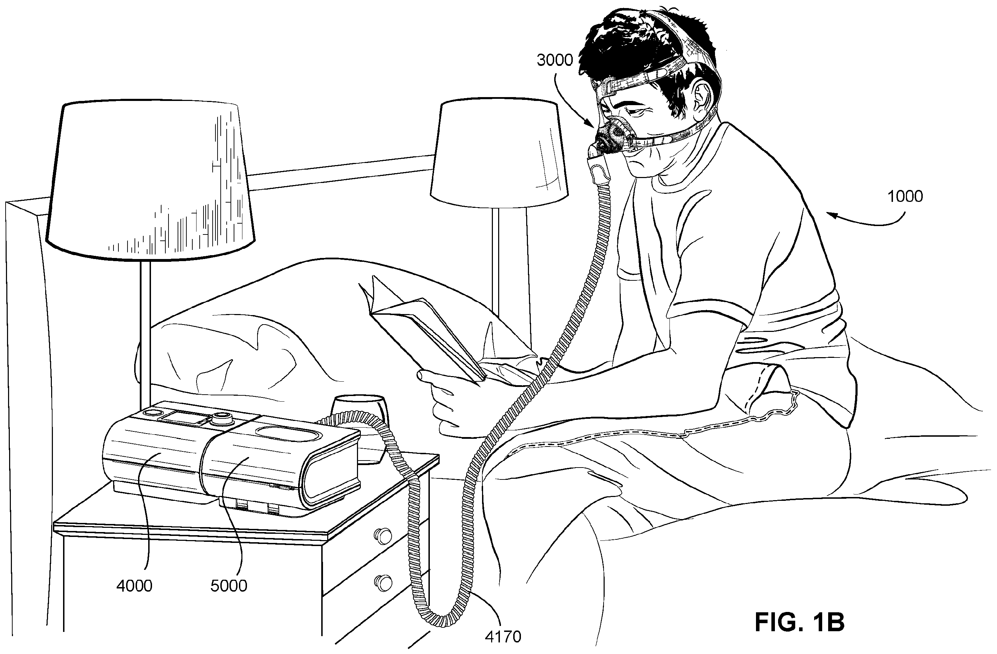

   Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

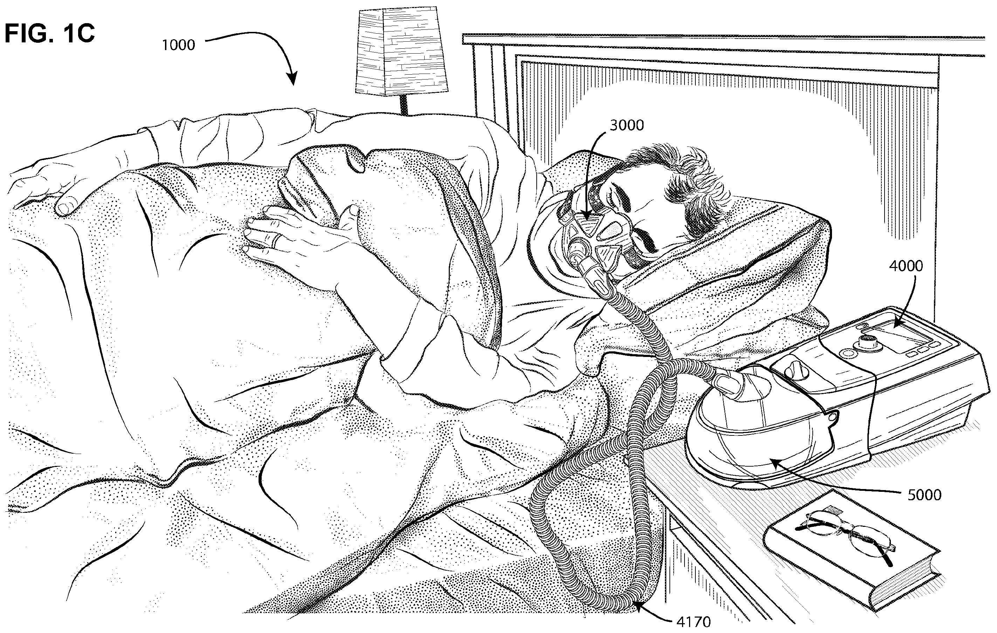

   Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.
3.2 RESPIRATORY SYSTEM AND FACIAL ANATOMY
   Fig. 2A shows an overview of a human respiratory system including the nasal and oral cavities, the larynx, vocal folds, oesophagus, trachea, bronchus, lung, alveolar sacs, heart and diaphragm.
3.3 PATIENT INTERFACE
   Fig. 3A shows a patient interface in the form of a nasal mask in accordance with one form of the present technology.
   Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
   Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
   Fig. 3D shows a schematic of a cross-section through a structure at a point. An outward normal at the point is indicated. The curvature at the point has a value of zero.
   Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
   Fig. 3F shows a schematic of a cross-section through a structure at a point. An outward normal at the point is indicated. The curvature at the point has a negative sign, and a relatively large magnitude when compared to the magnitude of the curvature shown in Fig. 3E.
   Fig. 3G shows a cushion for a mask that includes two pillows. An exterior surface of the cushion is indicated. An edge of the surface is indicated. Dome and saddle regions are indicated.
   Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.
   Fig. 3I shows the surface of a structure, with a one dimensional hole in the surface. The illustrated plane curve forms the boundary of a one dimensional hole.
   Fig. 3J shows a cross-section through the structure of Fig.3I. The illustrated surface bounds a two dimensional hole in the structure of Fig. 3I.
   Fig. 3K shows a perspective view of the structure of Fig. 3I, including the two dimensional hole and the one dimensional hole. Also shown is the surface that bounds a two dimensional hole in the structure of Fig. 3I.
   Fig. 3L shows a mask having an inflatable bladder as a cushion.
   Fig. 3M shows a cross-section through the mask of Fig. 3L, and shows the interior surface of the bladder. The interior surface bounds the two dimensional hole in the mask.
   Fig. 3N shows a further cross-section through the mask of Fig. 3L. The interior surface is also indicated.
   Fig. 3O illustrates a left-hand rule.
   Fig. 3P illustrates a right-hand rule.
   Fig. 3Q shows a left ear, including the left ear helix.
   Fig. 3R shows a right ear, including the right ear helix.
   Fig. 3S shows a right-hand helix.
   Fig. 3T shows a view of a mask, including the sign of the torsion of the space curve defined by the edge of the sealing membrane in different regions of the mask.
3.4 RPT DEVICE
   Fig. 4A shows an RPT device in accordance with one form of the present technology.

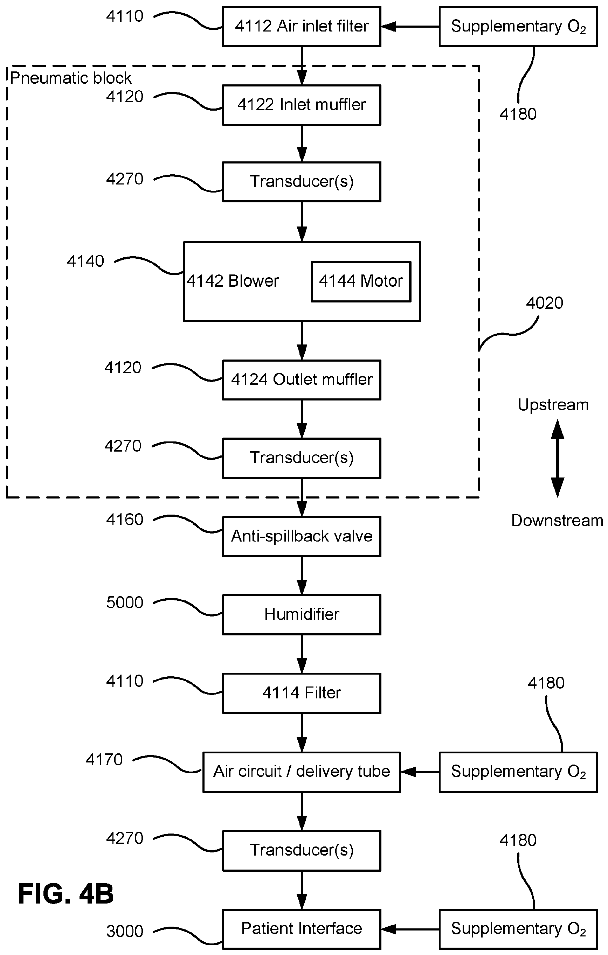

   Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

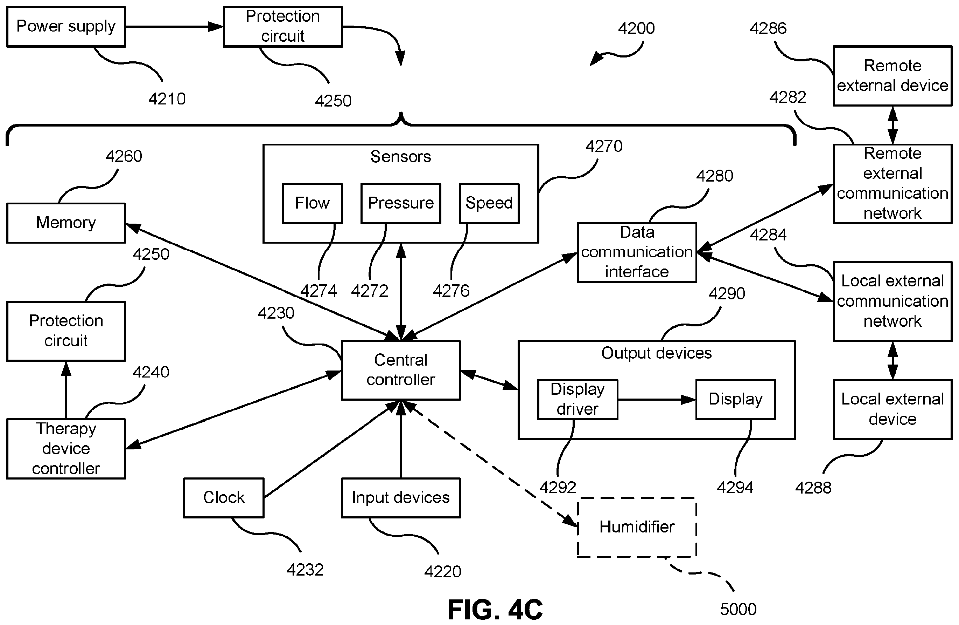

   Fig. 4C is a schematic diagram of the electrical components of an RPT device in accordance with one form of the present technology.
3.5 HUMIDIFIER
   Fig. 5A shows an isometric view of a humidifier in accordance with one form of the present technology.
   Fig. 5B shows an isometric view of a humidifier in accordance with one form of the present technology, showing a humidifier reservoir 5110 removed from the humidifier reservoir dock 5130
3.6 BREATHING WAVEFORMS
   Fig. 6A shows a model typical breath waveform of a person while sleeping.
3.7 AIR FILTRATION APPARATUS OF THE PRESENT TECHNOLOGY
   Fig. 7 is a front perspective view of an air filtration apparatus in accordance with one form of the technology.
   Fig. 8 is a rear perspective view of a HEPA filter.
   Fig. 9 is a rear perspective view of the air filtration apparatus of Fig 7.
   Fig. 10 is a top view of the filtration apparatus of Fig. 7, with a planar surface of an adjacent RPT device represented by plane R-R.
   Fig.11 is a side view of the air filtration apparatus of Fig, 7.
   Fig.12 is a front perspective view of the air filtration apparatus of Fig. 7 with the HEPA filter removed.
   Fig 13. is a top view an air filtration apparatus in accordance with another form of the technology.
   Fig.14 is block diagram of a system for providing patient information according to one form of the technology.

### 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

### 4.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 4.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000.

### 4.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

### 4.4 RPT DEVICE

An RPT device 4000 in accordance with one aspect of the present technology comprises mechanical, pneumatic, and/or electrical components and is configured to execute one or more algorithms, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH₂O, or at least 10cmH₂O, or at least 20 cmH₂O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors 4274.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

### 4.4.1 RPT device mechanical & pneumatic components

An RPT device may comprise one or more of the following components in an integral unit. In an alternative form, one or more of the following components may be located as respective separate units.

### 4.4.1.1 RPT device air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000.

### 4.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000.

### 4.4.1.3 Pressure generator

In one form of the present technology, a pressure generator 4140 for producing a flow, or a supply, of air at positive pressure is a controllable blower 4142. For example the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH₂O to about 20 cmH₂O, or in other forms up to about 30 cmH₂O when delivering respiratory pressure therapy. The blower may be as described in any one of the following patents or patent applications: U.S.

Patent No. 7,866,944; U.S. Patent No. 8,638,014; U.S. Patent No. 8,636,479; and PCT Patent Application Publication No. WO 2013/020167.

The pressure generator 4140 is under the control of the therapy device controller 4240.

In other forms, a pressure generator 4140 may be a piston-driven pump, a pressure regulator connected to a high pressure source (e.g. compressed air reservoir), or a bellows.

### 4.4.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000 .

In one form, a signal from a transducer 4270 may be filtered, such as by low-pass, high-pass or band-pass filtering.

### 4.4.1.4.1 Flow rate sensor

A flow rate sensor 4274 in accordance with the present technology may be based on a differential pressure transducer, for example, an SDP600 Series differential pressure transducer from SENSIRION.

In one form, a signal generated by the flow rate sensor 4274 and representing a flow rate is received by the central controller 4230.

### 4.4.1.4.2 Pressure sensor

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC.

In one form, a signal generated by the pressure sensor 4272 is received by the central controller 4230.

### 4.4.1.4.3 Motor speed transducer

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

### 4.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 4.4.2 RPT device electrical components

### 4.4.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

### 4.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form, the input device 4220 may be constructed and arranged to allow a person to select a value and/or a menu option.

### 4.4.2.3 Central controller

In one form of the present technology, the central controller 4230 is one or a plurality of processors suitable to control an RPT device 4000.

Suitable processors may include an x86 INTEL processor, a processor based on ARM^{®} Cortex^{®}-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.

In one form of the present technology, the central controller 4230 is a dedicated electronic circuit.

In one form, the central controller 4230 is an application-specific integrated circuit. In another form, the central controller 4230 comprises discrete electronic components.

The central controller 4230 may be configured to receive input signal(s) from one or more transducers 4270, one or more input devices 4220, and the humidifier 5000.

The central controller 4230 may be configured to provide output signal(s) to one or more of an output device 4290, a therapy device controller 4240, a data communication interface 4280, and the humidifier 5000.

In some forms of the present technology, the central controller 4230 is configured to implement the one or more methodologies described herein, such as the one or more algorithms expressed as computer programs stored in a non-transitory computer readable storage medium, such as memory 4260. In some forms of the present technology, the central controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein.

### 4.4.2.4 Clock

The RPT device 4000 may include a clock 4232 that is connected to the central controller 4230.

### 4.4.2.5 Therapy device controller

In one form of the present technology, therapy device controller 4240 is a therapy control module that forms part of the algorithms executed by the central controller 4230.

In one form of the present technology, therapy device controller 4240 is a dedicated motor control integrated circuit. For example, in one form a MC33035 brushless DC motor controller, manufactured by ONSEMI is used.

### 4.4.2.6 Protection circuits

The one or more protection circuits 4250 in accordance with the present technology may comprise an electrical protection circuit, a temperature and/or pressure safety circuit.

### 4.4.2.7 Memory

In accordance with one form of the present technology the RPT device 4000 includes memory 4260, e.g., non-volatile memory. In some forms, memory 4260 may include battery powered static RAM. In some forms, memory 4260 may include volatile RAM.

Memory 4260 may be located on the PCBA 4202. Memory 4260 may be in the form of EEPROM, or NAND flash.

Additionally or alternatively, RPT device 4000 includes a removable form of memory 4260, for example a memory card made in accordance with the Secure Digital (SD) standard.

In one form of the present technology, the memory 4260 acts as a non-transitory computer readable storage medium on which is stored computer program instructions expressing the one or more methodologies described herein, such as the one or more algorithms 4300.

### 4.4.2.8 Data communication systems

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor.

In one form, remote external communication network 4282 is the Internet. The data communication interface 4280 may use wired communication (e.g. via Ethernet, or optical fibre) or a wireless protocol (e.g. CDMA, GSM, LTE) to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.

The local external device 4288 may be a personal computer, mobile phone, tablet or remote control.

### 4.4.2.9 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

### 4.4.2.9.1 Display driver

A display driver 4292 receives as an input the characters, symbols, or images intended for display on the display 4294, and converts them to commands that cause the display 4294 to display those characters, symbols, or images.

### 4.4.2.9.2 Display

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

### 4.4.3 RPT device algorithms

As mentioned above, in some forms of the present technology, the central controller 4230 may be configured to implement one or more algorithms expressed as computer programs stored in a non-transitory computer readable storage medium, such as memory 4260. The algorithms are generally grouped into groups referred to as modules.

In other forms of the present technology, some portion or all of the algorithms may be implemented by a controller of an external device such as the local external device 4288 or the remote external device 4286. In such forms, data representing the input signals and / or intermediate algorithm outputs necessary for the portion of the algorithms to be executed at the external device may be communicated to the external device via the local external communication network 4284 or the remote external communication network 4282. In such forms, the portion of the algorithms to be executed at the external device may be expressed as computer programs stored in a non-transitory computer readable storage medium accessible to the controller of the external device. Such programs configure the controller of the external device to execute the portion of the algorithms.

### 4.5 AIR FILTRATION APPARATUS

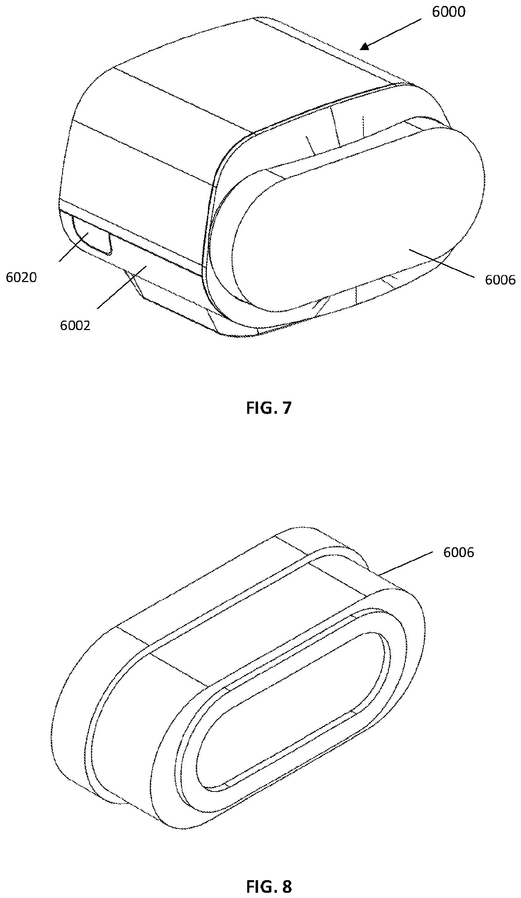

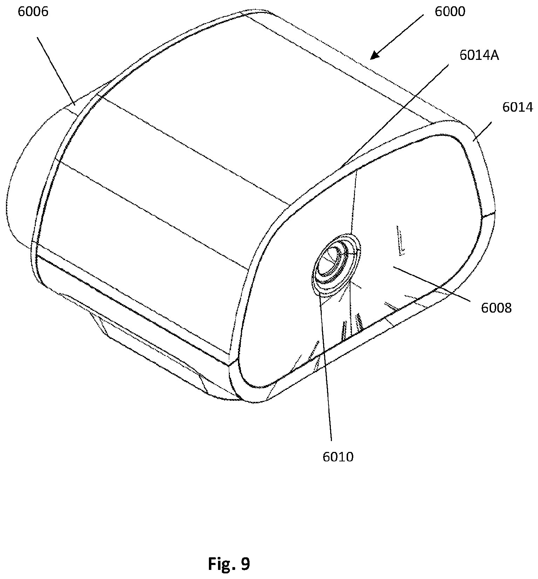

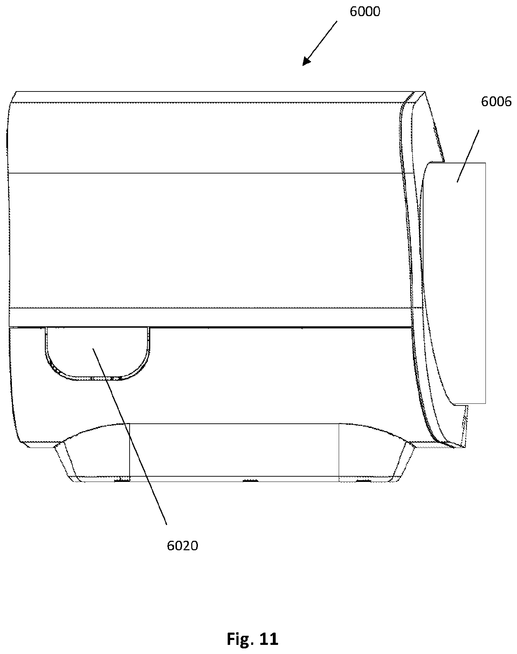

Referring to Figs. 7 to 12 and 14, an air filtration apparatus 6000 is shown. The apparatus 6000 comprises a housing 6002. A blower 6004 is provided within the housing 6002. In the example shown the blower 6004 comprises a motor and an impeller inside a volute, for example as described above with reference to a RPT device 4000, however, other forms of blower may be used. For example, in one example the blower may comprise a fan rather than an impeller.

The housing 6002 defines an inlet flow path 6026 (e.g. inlet pneumatic flow path) from ambient to an inlet of the blower 6004 and an outlet flow path 6028 (e.g. outlet pneumatic flow path) from an outlet of the blower 6004 to ambient. A filter means 6006 comprising a HEPA filter is provided in the inlet flow path.

The blower 6004 is configured to supply pressurised air to the outlet flow path at a flow rate of between 5 L/min and 300 L/min with the filter 6006 installed, at least when the filter is new. In examples where the apparatus is configured for use with a predetermined RPT device (described further below) the apparatus may be configured to have a flow rate of at least 80% of the maximum rated flow rate of the RPT device. In examples, the apparatus is configured to supply pressurised air to the outlet flow path at a flow rate of up to substantially 200 L/min.

In one example the filter 6006 may have an efficiency of at least 99% for particles 0.3µm or larger, for example around 99.97%.

In some examples the air filtration apparatus 6000 is configured for use with a predetermined RPT device, or at least for use with a range or group of RPT devices having a predetermined shape around their inlet ports. In examples the shape of the housing 6002 is configured to define a bypass flow path between an outer surface 6008 of the housing 6002 surrounding the outlet 6010 and the inlet of the RPT device. In examples a portion 6012 of the outer surface 6008 of the air filtration device, surrounding the outlet 6010, is non-planar. In the example shown in Figs. 7-12, the portion 6012 of the surface 6008 surrounding the outlet 6010 has two non-zero principal curvatures, that is, the surface comprises at least one dome region, for example a concave dome region.

In examples, an outer perimeter 6014 of the outer surface 6008 of the air filtration device, surrounding the outlet 6010, has at least one portion 6014A which is not co-planar with the remainder of the outer perimeter.

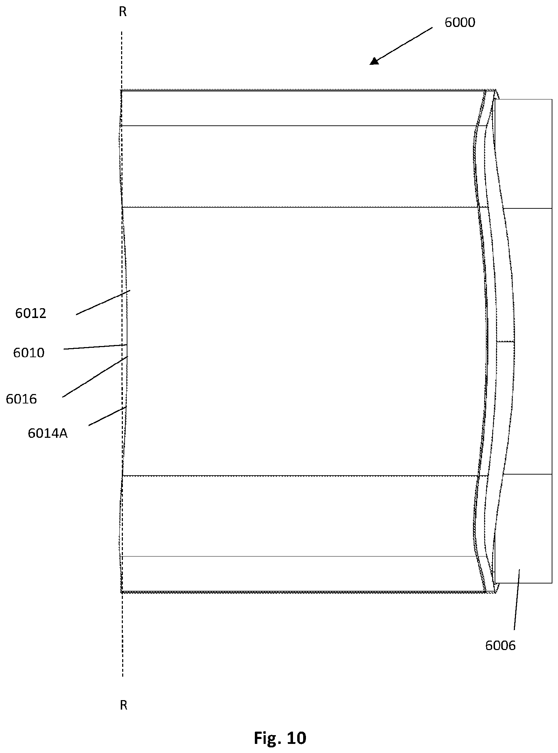

As shown in Figure 10, the non-coplanar portion 6014A and non-planar outer surface portion 6012 create a clearance space 6016 when the filtration apparatus 6000 is positioned immediately adjacent an RPT with a planar face surrounding the inlet to the RPT, the planar face represented by axis R-R in Fig. 10. The clearance space 6016 allows ambient air to be drawn in to the inlet of the RPT if the flow rate of the filtration apparatus 6000 is not sufficient to supply all of the RPT's flow requirement, for example if the blower of the air filtration apparatus is not operating (for example in the case of a malfunction or power cut).

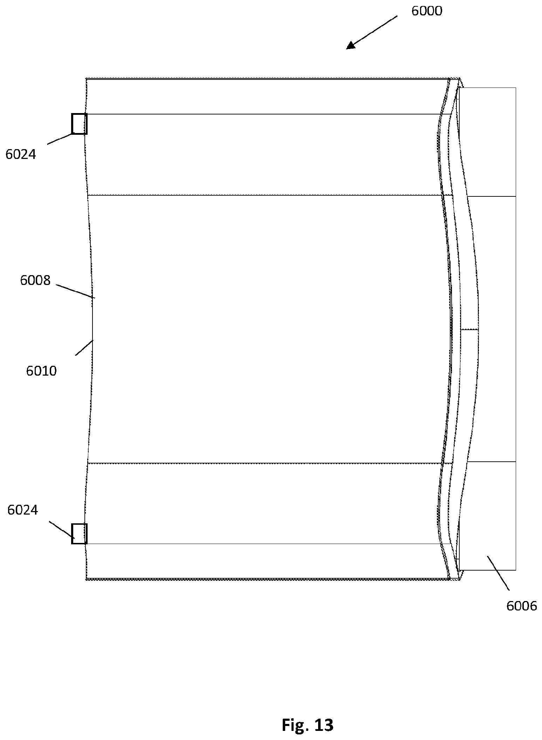

In some examples the air filtration apparatus 6000 may be configured for use with an RPT device which has a non-planar surface surrounding its inlet. In such examples the surface of the apparatus 6000 around the outlet 6010 may be planar while still providing the required clearance space for the bypass flow path. As shown in Fig. 13, in examples the apparatus 6000 may comprise one or more spacer portions, e.g. stand-offs 6024, configured to ensure that the surface 6008 of the apparatus 6000 surrounding the outlet 6010 cannot be placed immediately adjacent a surface of an adjacent RPT device.

In other examples the air filtration apparatus 6000 may comprise one or more apertures and/or conduits which allow ambient air to enter a flow path between the outlet of the blower and the outlet 6010. In such examples, one or more one way valves (e.g. similar to anti asphyxiation valves) may be provided to allow ambient air to enter the flow path between the outlet of the blower and the outlet 6010, if required, but to substantially prevent escape of pressurised air when the blower is operating correctly. In some examples, the outlet 6010 may be sealed to an inlet of the RPT device 4000, such that no ambient air can enter the inlet of the RPT device except through the specific apertures/flow paths provided. In examples, the air filtration apparatus 6000 may be connectable to the RPT device 4000.

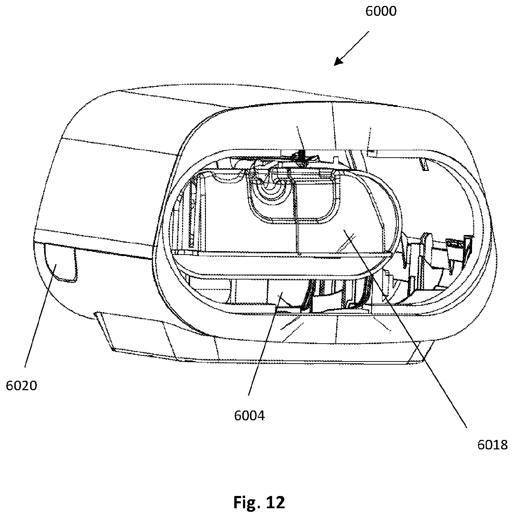

As shown in Fig. 12, the housing 6002 may comprise a baffle 6018 in the inlet flow path provided between an inlet to the blower 6004 and the filter 6006. The baffle 6018 may ensure that the filter medium does not block the inlet to the blower 6004 and/or may be configured to reduce noise emitted from the apparatus 6000 though the inlet flow path.

The filtration apparatus 6000 may comprise an air quality sensor 6020 mounted so as to be in contact with the ambient air. In one example the sensor 6020 may be a PM 2.5 sensor. In examples, an air quality sensor 6020 may additionally or alternatively be mounted downstream of the filter means 6006, for example in the inlet flow path and/or the outlet flow path.

The apparatus 6000 may also comprise a data communication system substantially as described above, for communication of data, for example data including an indication of air quality (e.g. the concentration of particulates in the ambient air), an indication of the quality of the air supplied to the patient, and/or the flow rate or air through the apparatus, to a remote external communication network 4282 and/or a local external communication network 4284, as described further below.

The filtration apparatus 6000 may be mains powered, battery powered or may be primarily mains powered with a backup battery.

Because the filtration apparatus 6000 comprises its own blower, the apparatus may be used with RPT devices, for example one or more of the RPT devices of the prior art, which would not be capable of supplying air to a patient at a full range of therapeutic pressures and flow rates if a HEPA filter was added directly to the inlet flow path of the RPT device.

In examples, the apparatus 6000 may have similar dimensions to the predetermined RPT device (for example ResMed's Airsense 11 ^{™}). For example, at least one of the height, width and length of the apparatus 6000 may be within 10% of that of the predetermined RPT device. In examples, both the height and width of the apparatus 6000 may be within 10% of those of the predetermined RPT device. In another example the height, length and width dimensions of the apparatus 6000 may be within 10% of the corresponding dimensions of the predetermined RPT device. In examples, the apparatus may have a height of less than 150 mm and/or a length of less than 285 mm and/or a width of less than 105 mm. This may ensure that the apparatus 6000 can conveniently be located, in use, in an operative position adjacent the RPT device (e.g. on a bedside table).

In examples, the outlet 6010 of the apparatus 6000 may be configured to substantially align with an inlet to a predetermined RPT. For example, a centre of the outlet 6010 may be between 50mm -100mm high.

### 4.5.1 Alternative examples

In alternative examples the filter means may be configured to remove volatile organic compounds (VOCs) from the air. The HEPA filter described above may be replaced with a suitable VOC filter element, for example comprising activated charcoal or activated carbon. In such embodiments, the air quality sensor 6020 may be configured to measure VOC concentration.

In other examples a combination of filters may be used, for example HEPA and VOC (or a single filter which is able to perform both functions), or a VOC filter in combination with a coarser (than HEPA) particulate filter. In such embodiments, air quality sensor(s) 6020 may be provided to measure both VOC concentration and particulates, or only one of those.

In examples the air filtration device may also be configured to modify the temperature and/or humidity of the air, in addition to filtering the air as described above.

### 4.6 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349, which is incorporated herewithin in its entirety by reference.

### 4.6.1 Supplementary gas delivery

In one form of the present technology, supplementary gas, e.g. oxygen, 4180 is delivered to one or more points in the pneumatic path, such as upstream of the pneumatic block 4020, to the air circuit 4170, and/or to the patient interface 3000.

### 4.7 HUMIDIFIER

### 4.7.1 Humidifier overview

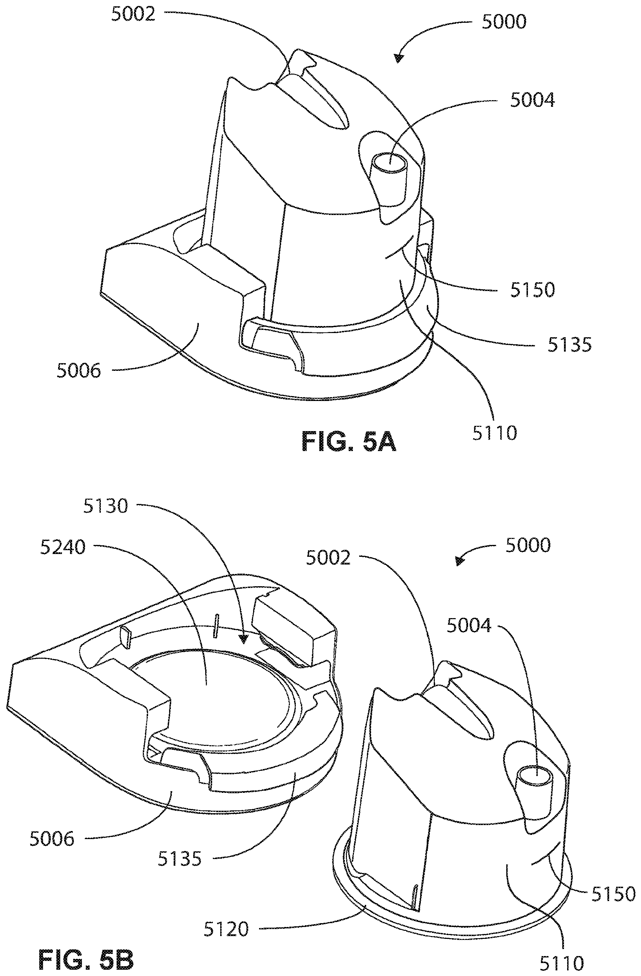

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 4.8 BREATHING WAVEFORMS

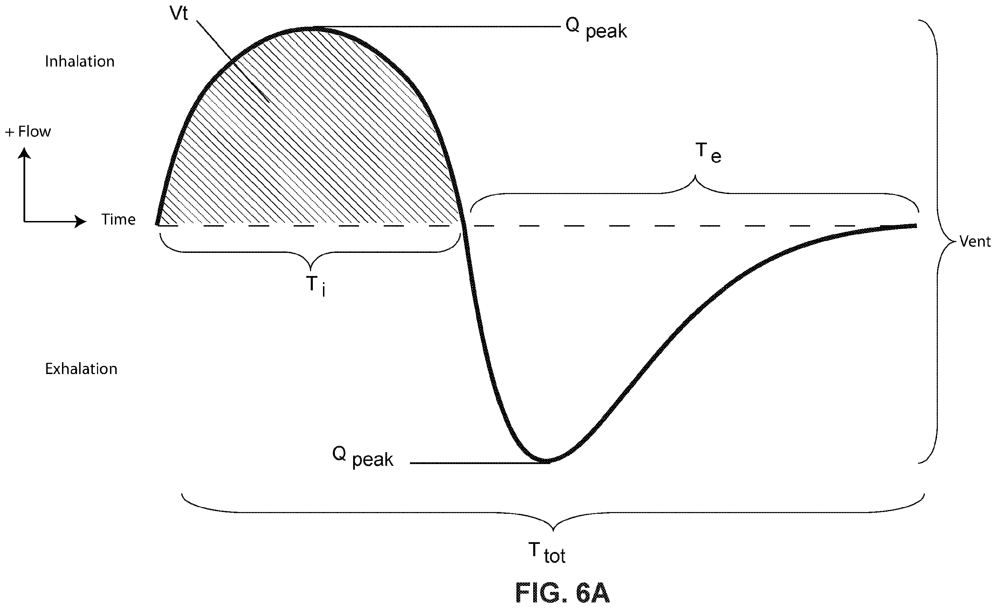

Fig. 6A shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak* -0.5 L/s. The total duration of the breath, *Ttot*, is about 4s. The person typically breathes at a rate of about 15 breaths per minute (BPM), with Ventilation *Vent* about 7.5 L/min. A typical duty cycle, the ratio of *Ti* to *Ttot*, is about 40%.

### 4.9 MONITORING SYSTEM

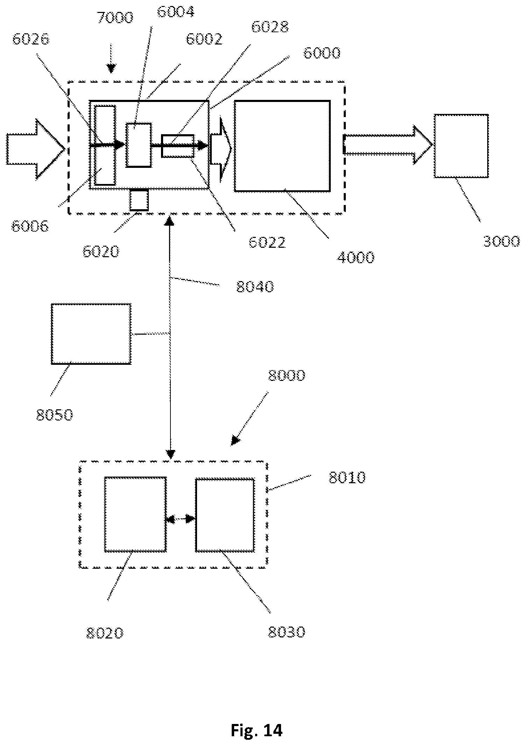

In one form of the technology a system 8000 for providing patient information related to the patient's respiratory pressure therapy comprises a data server 8010 comprising one or more processors 8020, at least one memory 8030, as shown in Fig. 14, and other components typically present in such computing environments. The processing capabilities of the processor 8020 may be provided, for example, by one or more general-purpose processors, one or more special-purpose processors, or cloud computing services providing access to a shared pool of computing resources configured in accordance with desired characteristics, service models, and deployment models.

In the example illustrated, the memory 8030 stores information accessible by processor 8020, the information including instructions that may be executed by the processor 8020 and data that may be retrieved, manipulated or stored by the processor 8020. The memory 8030 may be of any suitable means known in the art, capable of storing information in a manner accessible by the processor 8020, including a computer readable medium, or other medium that stores data that may be read with the aid of an electronic device. Although the processor 8020 and memory 8030 are each illustrated as being within a single unit, it should be appreciated that this is not intended to be limiting, and that the functionality of each as herein described may be performed by multiple processors and memories, that may or may not be remote from each other and the remainder of system 8000.

The instructions may include any set of instructions suitable for execution by the processor 8020. For example, the instructions may be stored as computer code on the computer readable medium. The instructions may be stored in any suitable computer language or format. Data may be retrieved, stored or modified by processor 8020 in accordance with the instructions. The data may also be formatted in any suitable computer readable format. The data may be contained at a single location, or may alternatively be stored in multiple memories or locations. The data may include one or more databases.

The system 8000 is configured to receive data from a system 7000 for providing air to a patient at positive pressure for respiratory therapy. The data may be transmitted to the data server 8010 over a network 8040.

The data comprises data from an air quality sensor 6020 associated with the system 7000 for providing air to a patient at positive pressure for respiratory therapy, for example a sensor 6020 which is part of a filtration apparatus 6000. The data may also comprise settings of the RPT device 4000, and / or data describing one or more of the variables of the therapy, e.g. flow rate, treatment pressure, and leak flow rate, over the therapy session. In examples the system 7000 comprises two air quality sensors 6020, 6022, one sensor 6020 positioned for measuring particulates (and/or other contaminants) in the ambient air, and one sensor 6022 positioned for measuring particulates (and/or other contaminants) in air which has been filtered by the system (for example by a HEPA filter, as described above) before the air is supplied to the patient interface 3000.

The data server 8010 may be configured to receive therapy data from the RPT device 4000 and to compute therapy summary data for the therapy session from the therapy data, as described in PCT/US2015/043266. The therapy summary data additionally or alternatively comprises data from one or both air quality sensors 6020, 6022 and/or data related to a difference between the readings from the air quality sensors 6020, 6022 (e.g. the reduction in particulate levels in the air due to the filter).

The system 8000 may also contain a patient computing device 8050 associated with the patient, connected to the network 8040. The patient computing device 8050 may be a personal computer, mobile phone, tablet computer, or other device.

In an implementation of the patient management system (not shown), the system 7000 for providing air to a patient at positive pressure for respiratory therapy is configured to communicate with the patient computing device 8050 via a local wired network or a wireless network (not shown) based on a protocol such as Bluetooth or WiFi. In one implementation, the patient computing device 8050 intermediates between the system 7000 for providing air to a patient at positive pressure for respiratory therapy and the remotely located entities of the patient management system over the network 8040. In another example the patient computing device performs the function of the server 8010.

### 4.10 PORTABLE OXYGEN CONCENTRATOR

Oxygen concentrators may take advantage of pressure swing adsorption (PSA). Pressure swing adsorption may involve using a compressor to increase gas pressure inside a canister that contains particles of a gas separation adsorbent. As the pressure increases, certain molecules in the gas may become adsorbed onto the gas separation adsorbent. Removal of a portion of the gas in the canister under the pressurized conditions allows separation of the non-adsorbed molecules from the adsorbed molecules. The gas separation adsorbent may be regenerated by reducing the pressure, which reverses the adsorption of molecules from the adsorbent. Further details regarding oxygen concentrators may be found, for example, in U.S. Published Patent Application No. 2009-0065007, published March 12, 2009, and entitled "Oxygen Concentrator Apparatus and Method".

Ambient air usually includes approximately 78% nitrogen and 21% oxygen with the balance comprised of argon, carbon dioxide, water vapor and other trace gases. If a gas mixture such as air, for example, is passed under pressure through a vessel containing a gas separation adsorbent bed that attracts nitrogen more strongly than it does oxygen, part or all of the nitrogen will stay in the bed, and the gas coming out of the vessel will be enriched in oxygen. When the bed reaches the end of its capacity to adsorb nitrogen, it can be regenerated by reducing the pressure, thereby releasing the adsorbed nitrogen. It is then ready for another cycle of producing oxygen enriched gas. By alternating canisters in a two-canister system, one canister can be collecting oxygen while the other canister is being purged (resulting in a continuous separation of the oxygen from the nitrogen). In this manner, oxygen can be accumulated, such as in a storage container or other pressurizable vessel or conduit coupled to the canisters, from the ambient air for a variety of uses include providing supplemental oxygen to patients.

### 4.11 RESPIRATORY THERAPY MODES

Various respiratory therapy modes may be implemented by the disclosed respiratory therapy system including CPAP therapy, bilevel therapy and/or high flow therapy.

### 4.12 GLOSSARY

For the purposes of the present technology disclosure, in certain forms of the present technology, one or more of the following definitions may apply. In other forms of the present technology, alternative definitions may apply.

### 4.12.1 General

*Air*: In certain forms of the present technology, air may be taken to mean atmospheric air, and in other forms of the present technology air may be taken to mean some other combination of breathable gases, e.g. atmospheric air enriched with oxygen.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy*: CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy*: Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate*: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd*, is the flow rate of air leaving the RPT device. Total flow rate, *Qt*, is the flow rate of air and any supplementary gas reaching the patient interface via the air circuit. Vent flow rate, *Qv*, is the flow rate of air leaving a vent to allow washout of exhaled gases. Leak flow rate, *Ql*, is the flow rate of leak from a patient interface system or elsewhere. Respiratory flow rate, *Qr*, is the flow rate of air that is received into the patient's respiratory system.

*Flow therapy*: Respiratory therapy comprising the delivery of a flow of air to an entrance to the airways at a controlled flow rate referred to as the treatment flow rate that is typically positive throughout the patient's breathing cycle.

*Humidifier*: The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak*: The word leak will be taken to be an unintended flow of air. In one example, leak may occur as the result of an incomplete seal between a mask and a patient's face. In another example leak may occur in a swivel elbow to the ambient.

*Noise, conducted (acoustic)*: Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise*, *radiated (acoustic)*: Radiated noise in the present document refers to noise which is carried to the patient by the ambient air. In one form, radiated noise may be quantified by measuring sound power/pressure levels of the object in question according to ISO 3744.

*Noise*, *vent (acoustic)*: Vent noise in the present document refers to noise which is generated by the flow of air through any vents such as vent holes of the patient interface.

*Patient*: A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm*, while the treatment pressure, which represents a target value to be achieved by the interface pressure *Pm* at the current instant of time, is given the symbol *Pt.*

*Respiratory Pressure Therapy (RPT)*: The application of a supply of air to an entrance to the airways at a treatment pressure that is typically positive with respect to atmosphere.

*Ventilator*: A mechanical device that provides pressure support to a patient to perform some or all of the work of breathing.

### 4.12.1.1 Materials

*Silicone or Silicone Elastomer*: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate*: a thermoplastic polymer of Bisphenol-A Carbonate.

### 4.12.1.2 Mechanical properties

*Resilience*: Ability of a material to absorb energy when deformed elastically and to release the energy upon unloading.

*Resilient*: Will release substantially all of the energy when unloaded. Includes e.g. certain silicones, and thermoplastic elastomers.

*Hardness*: The ability of a material *per se* to resist deformation (e.g. described by a Young's Modulus, or an indentation *hardness* scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component*: The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component*: A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 4.12.2 Respiratory cycle

*Apnea*: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate*: The rate of spontaneous respiration of a patient, usually measured in breaths per minute.

*Duty cycle*: The ratio of inhalation time, *Ti* to total breath time, *Ttot.*

*Effort (breathing)*: The work done by a spontaneously breathing person attempting to breathe.

*Expiratory portion of a breathing cycle:* The period from the start of expiratory flow to the start of inspiratory flow.

*Flow limitation*: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) *Flattened*: Having a rise followed by a relatively flat portion, followed by a fall.
(ii) *M-shaped*: Having two local peaks, one at the leading edge, and one at the trailing edge, and a relatively flat portion between the two peaks.
(iii) *Chair-shaped*: Having a single local peak, the peak being at the leading edge, followed by a relatively flat portion.
(iv) *Reverse-chair shaped*: Having a relatively flat portion followed by single local peak, the peak being at the trailing edge.

*Hypopnea*: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea*: An increase in flow to a level higher than normal.

*Inspiratory portion of a breathing cycle*: The period from the start of inspiratory flow to the start of expiratory flow will be taken to be the inspiratory portion of a breathing cycle.

*Patency (airway)*: The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP)*: The pressure above atmosphere in the lungs that exists at the end of expiration.

*Peak flow rate (Qpeak)*: The maximum value of flow rate during the inspiratory portion of the respiratory flow waveform.

*Respiratory flow rate*, patient *airflow rate*, *respiratory airflow rate (Qr)*: These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt)*: The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti)*: The duration of the inspiratory portion of the respiratory flow rate waveform.

*(exhalation) Time (Te)*: The duration of the expiratory portion of the respiratory flow rate waveform.

*(total) Time (Ttot)*: The total duration between the start of one inspiratory portion of a respiratory flow rate waveform and the start of the following inspiratory portion of the respiratory flow rate waveform.

*Typical recent ventilation*: The value of ventilation around which recent values of ventilation *Vent* over some predetermined timescale tend to cluster, that is, a measure of the central tendency of the recent values of ventilation.

*Upper airway obstruction (UAO)*: includes both partial and total upper airway obstruction. This may be associated with a state of flow limitation, in which the flow rate increases only slightly or may even decrease as the pressure difference across the upper airway increases (Starling resistor behaviour).

*Ventilation (Vent)*: A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 4.12.3 Ventilation

*Adaptive Servo-Ventilator (ASV)*: A servo-ventilator that has a changeable, rather than fixed target ventilation. The changeable target ventilation may be learned from some characteristic of the patient, for example, a respiratory characteristic of the patient.

*Backup rate*: A parameter of a ventilator that establishes the minimum breathing rate (typically in number of breaths per minute) that the ventilator will deliver to the patient, if not triggered by spontaneous respiratory effort.

*Cycled*: The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP)*: a base pressure, to which a pressure varying within the breath is added to produce the desired interface pressure which the ventilator will attempt to achieve at a given time.

*End expiratory pressure (EEP)*: Desired interface pressure which the ventilator will attempt to achieve at the end of the expiratory portion of the breath. If the pressure waveform template Π(Φ) is zero-valued at the end of expiration, i.e. Π(Φ) = 0 when Φ = 1, the EEP is equal to the EPAP.

*Inspiratory positive airway pressure (IPAP)*: Maximum desired interface pressure which the ventilator will attempt to achieve during the inspiratory portion of the breath.

*Pressure support*: A number that is indicative of the increase in pressure during ventilator inspiration over that during ventilator expiration, and generally means the difference in pressure between the maximum value during inspiration and the base pressure (e.g., *PS = IPAP - EPAP*). In some contexts pressure support means the difference which the ventilator aims to achieve, rather than what it actually achieves.

*Servo-ventilator*: A ventilator that measures patient ventilation, has a target ventilation, and which adjusts the level of pressure support to bring the patient ventilation towards the target ventilation.

*Spontaneous*/*Timed (S*/*T)*: A mode of a ventilator or other device that attempts to detect the initiation of a breath of a spontaneously breathing patient. If however, the device is unable to detect a breath within a predetermined period of time, the device will automatically initiate delivery of the breath.

*Swing*: Equivalent term to pressure support.

*Triggered*: When a ventilator delivers a breath of *air* to a spontaneously breathing patient, it is said to be triggered to do so at the initiation of the respiratory portion of the breathing cycle by the patient's efforts.

### 4.12.4 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

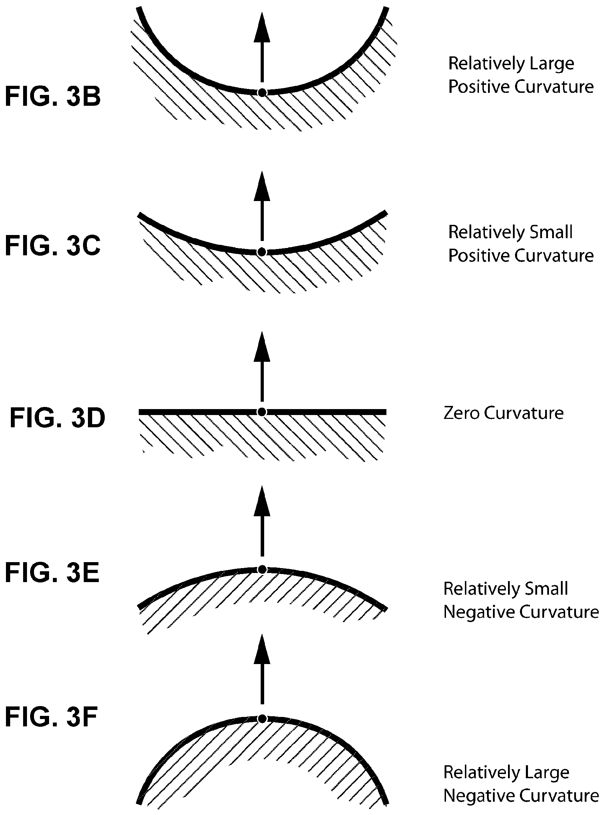

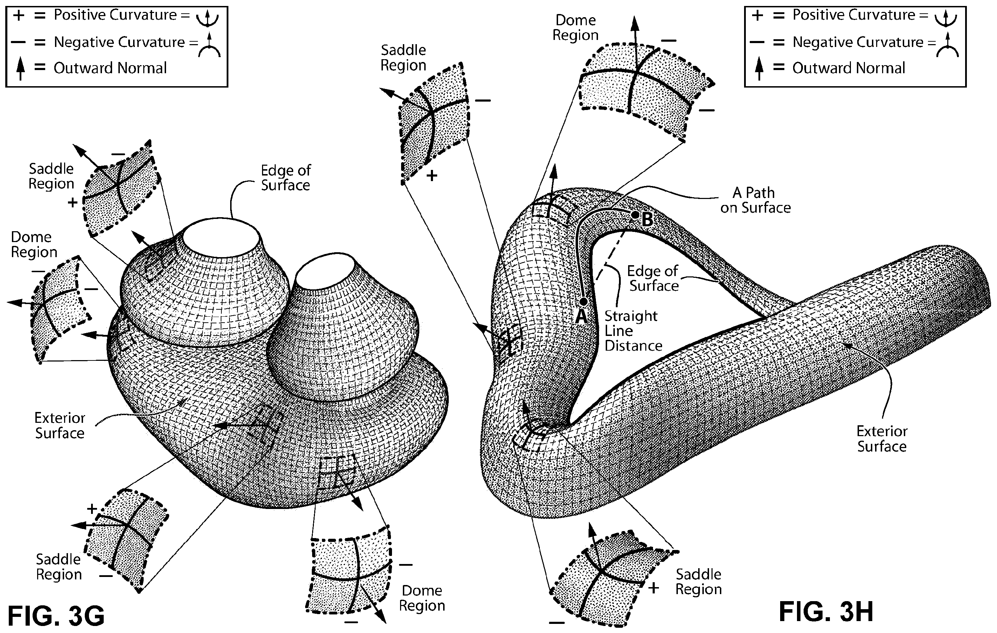

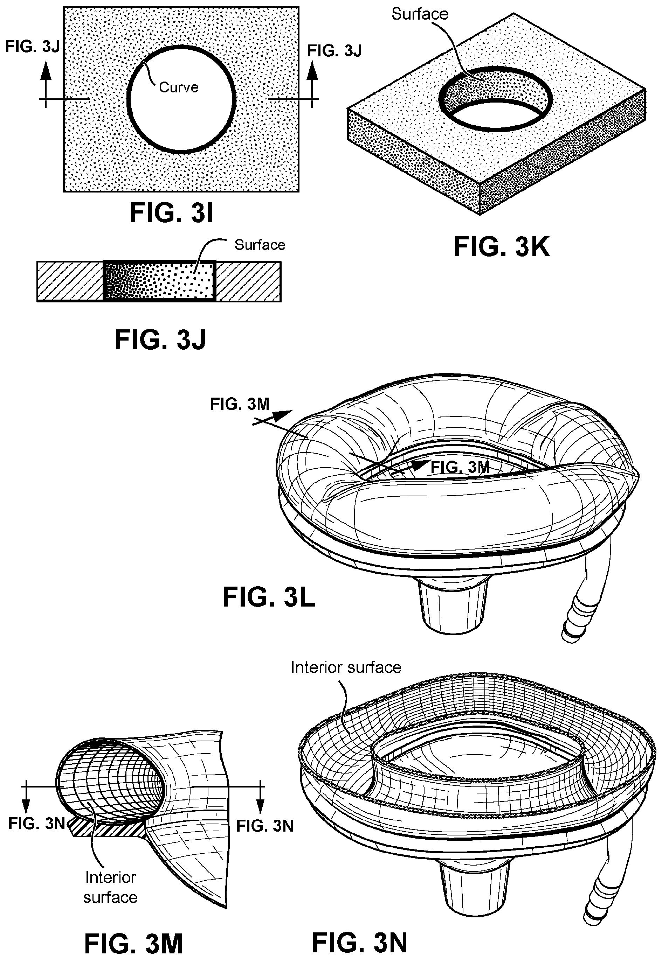

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 4.12.4.1 Curvature in one dimension

The curvature of a plane curve at *p* may be described as having a sign (e.g. positive, negative) and a magnitude (e.g. 1/radius of a circle that just touches the curve at *p*)*.*

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 4.12.4.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from *f*(0) to *f*(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 4.12.4.3 Space curves

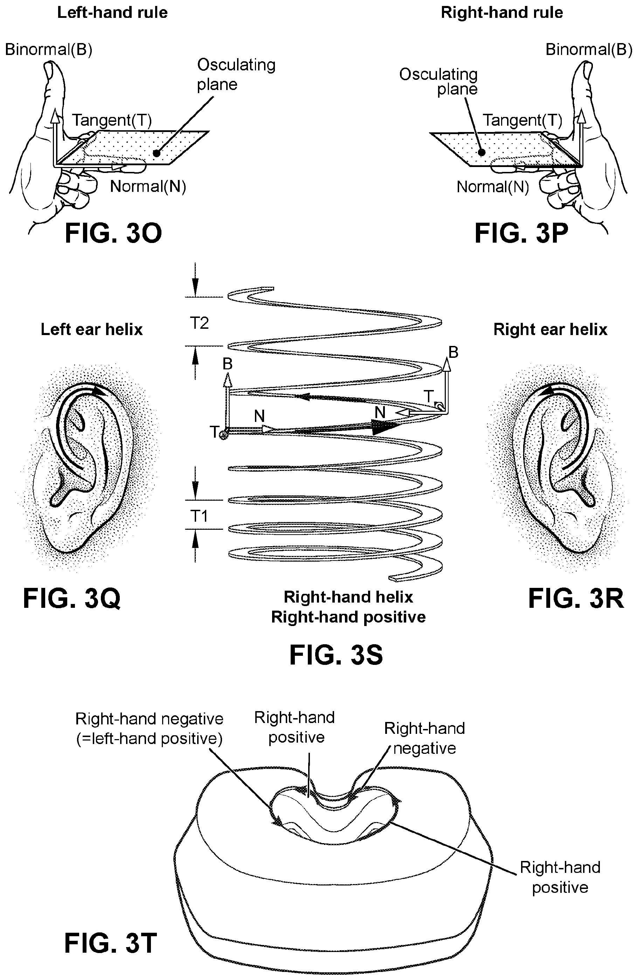

*Space curves*: Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

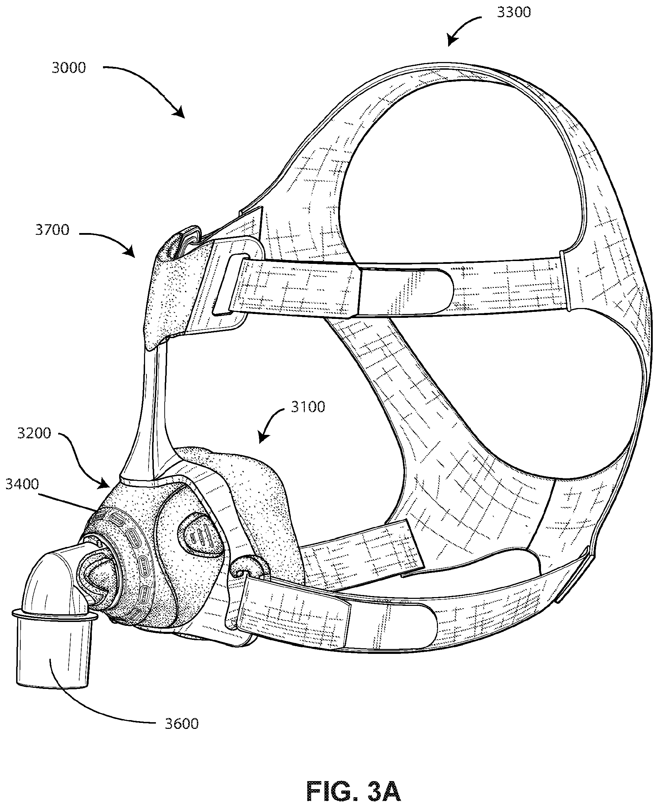

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 4.12.4.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 4.13 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the appended claims.

### 4.14 REFERENCE SIGNS LIST

| | |
|---|---|
| patient | 1000 |
| bed partner | 1100 |
| patient interface | 3000 |
| seal forming structure | 3100 |
| plenum chamber | 3200 |
| chord | 3210 |
| superior point | 3220 |
| inferior point | 3230 |
| positioning and stabilising structure | 3300 |
| vent | 3400 |
| connection port | 3600 |
| forehead support | 3700 |
| patient interface | 3800 |
| RPT device | 4000 |
| external housing | 4010 |
| upper portion | 4012 |
| lower portion | 4014 |
| panel | 4015 |
| chassis | 4016 |
| handle | 4018 |
| pneumatic block | 4020 |
| air filter | 4110 |
| inlet air filter | 4112 |
| outlet air filter | 4114 |
| muffler | 4120 |
| inlet muffler | 4122 |
| outlet muffler | 4124 |
| pressure generator | 4140 |
| blower | 4142 |
| motor | 4144 |
| anti-spillback valve | 4160 |
| air circuit | 4170 |
| supplementary gas | 4180 |
| electrical components | 4200 |
| PCBA | 4202 |
| electrical power supply | 4210 |
| input device | 4220 |
| central controller | 4230 |
| clock | 4232 |
| therapy device controller | 4240 |
| protection circuit | 4250 |
| memory | 4260 |
| transducers | 4270 |
| pressure sensor | 4272 |
| flow rate sensor | 4274 |
| motor speed transducer | 4276 |
| | |
| data communication interface | 4280 |
| remote external communication network | 4282 |
| local external communication network | 4284 |
| remote external device | 4286 |
| local external device | 4288 |
| output device | 4290 |
| display driver | 4292 |
| display | 4294 |
| humidifier | 5000 |
| humidifier inlet | 5002 |
| humidifier outlet | 5004 |
| humidifier base | 5006 |
| humidifier reservoir | 5110 |
| conductive portion | 5120 |
| reservoir dock | 5130 |
| locking lever | 5135 |
| water level indicator | 5150 |
| heating element | 5240 |
| air filtration apparatus | 6000 |
| housing | 6002 |
| blower | 6004 |
| HEPA filter | 6006 |
| outer surface | 6008 |
| outlet | 6010 |
| non-planar portion | 6012 |
| outer perimeter | 6014 |
| outer perimeter portion | 6014A |
| clearance space | 6016 |
| baffle | 6018 |
| air quality sensor | 6020 |
| particulate sensor | 6022 |
| stand off | 6024 |
| inlet flow path | 6026 |
| outlet flow path | 6028 |
| system for providing air to a patient | 7000 |
| system for providing patient information | 8000 |
| server | 8010 |
| processor | 8020 |
| memory | 8030 |
| network | 8040 |
| personal computing device | 8050 |

## Claims

1. A system (7000) for providing air to a patient at positive pressure for respiratory therapy, the system (7000) comprising an respiratory pressure therapy - RPT - device (4000) having an inlet and a predetermined shape around the inlet, the system (7000) further comprising an air filtration apparatus (6000) for supplying a flow of filtered air to the inlet of the RPT device (4000), the air filtration apparatus (6000) comprising an inlet flow path (6026), an outlet flow path (6028), an air outlet (6010), and a first blower (6004) configured to receive air from the inlet flow path (6026) and to supply air at above ambient pressure to the outlet flow path (6028), wherein the inlet flow path (6026) comprises a filter means comprising a HEPA filter (6006) and/or a VOC filter, and wherein the first blower (6004) is configured to supply pressurised air to the outlet flow path (6028) at a flow rate of at least 5 L/min,
wherein an air outlet (6010) of the air filtration apparatus (6000) is located relative to the air inlet of the RPT device (4000) such that the air inlet of the RPT device (4000) receives filtered air from the air outlet (6010) of the air filtration apparatus (6000),
wherein a shape of a housing (6002) of the air filtration apparatus (6000) is configured to define a bypass flow path between an outer surface (6008) of the housing (6002) surrounding the air outlet (6010) of the air filtration apparatus (6000) and the inlet of the RPT device (4000), which allows ambient air to flow to the inlet of the RPT device (4000) if the flow rate of the air filtration apparatus (6000) is not sufficient to supply all of the RPT device's (4000) flow requirement, and
wherein the RPT device (4000) further comprises a second blower.

2. The system (7000) of claim 1 comprising a HEPA filter (6006), wherein the HEPA filter (6006) is configured to have an efficiency of at least 99% for particles 0.3µm or larger.

3. The system (7000) of claim 1 or 2, wherein a portion of a surface of the apparatus surrounding the air outlet (6010) is non-planar.

4. The system (7000) of any one of claims 1 to 3, comprising one more spacer portions (6024) which protrude from a surface portion surrounding the air outlet (6010) of the air filtration apparatus (6000).

5. The system (7000) of any one of claims 1 to 4, comprising an air quality sensor (6020, 6022).

6. The system (7000) of claim 5, wherein the air quality sensor (6020, 6022) is configured to measure particulate levels in the ambient air surrounding the apparatus.

7. The system (7000) of claim 6, wherein the apparatus comprises a further air quality sensor (6022) downstream of the filter means.

8. The system (7000) of any one of claims 1 to 7, comprising a data communication system.

9. The system (7000) of claim 8, wherein the data communication system is a wireless data communication system.

10. The system (7000) of claim 8 or 9, wherein the data communication system communicates data related to one or more of: a flow rate through the apparatus; a level of particulates and/or VOCs in the ambient air; and a level of particulates and/or VOCs downstream of the HEPA filter (6006).

11. The system (7000) of any one of claims 1 to 10, wherein the bypass flow path is configured to allow sufficient bypass flow for the RPT device (4000) to provide an outlet air flow at a therapeutic pressure and flow rate.

12. The system (7000) of any one of claims 1 to 11, wherein the air filtration apparatus (6000) comprises a non-planar surface portion surrounding the air outlet (6010) of the air filtration apparatus (6000).

## Patentansprüche

1. System (7000) zum Bereitstellen von Luft mit Überdruck an einen Patienten zur Atemtherapie, wobei das System (7000) eine Atemdrucktherapie- (RPT-)Vorrichtung (4000) mit einem Einlass und einer vorbestimmten Form um den Einlass herum aufweist, wobei das System (7000) ferner eine Luftfilterungsvorrichtung (6000) zum Zuführen eines Stroms gefilterter Luft zum Einlass der RPT-Vorrichtung (4000) aufweist, wobei die Luftfilterungsvorrichtung (6000) einen Einlassströmungsweg (6026), einen Auslassströmungsweg (6028), einen Luftauslass (6010) und ein erstes Gebläse (6004) aufweist, das zum Aufnehmen von Luft aus dem Einlassströmungsweg (6026) und zum Zuführen von Luft mit einem Druck über dem Umgebungsdruck zu dem Auslassströmungsweg (6028) konfiguriert ist, wobei der Einlassströmungsweg (6026) eine Filtereinrichtung aufweist, die einen HEPA-Filter (6006) und/oder einen VOC-Filter aufweist, und wobei das erste Gebläse (6004) zum Zuführen von Druckluft zu dem Auslassströmungsweg (6028) mit einer Durchflussrate von mindestens 5 l/min konfiguriert ist,
wobei ein Luftauslass (6010) der Luftfilterungsvorrichtung (6000) derart relativ zum Lufteinlass der RPT-Vorrichtung (4000) angeordnet ist, dass der Lufteinlass der RPT-Vorrichtung (4000) gefilterte Luft vom Luftauslass (6010) der Luftfilterungsvorrichtung (6000) aufnimmt,
wobei eine Form eines Gehäuses (6002) der Luftfilterungsvorrichtung (6000) zum Definieren eines Bypass-Strömungswegs zwischen einer Außenfläche (6008) des Gehäuses (6002), die den Luftauslass (6010) der Luftfilterungsvorrichtung (6000) umgibt, und dem Einlass der RPT-Vorrichtung (4000) konfiguriert ist, der es ermöglicht, dass Umgebungsluft zum Einlass der RPT-Vorrichtung (4000) strömt, wenn die Durchflussrate der Luftfilterungsvorrichtung (6000) nicht ausreicht, um den gesamten Strömungsbedarf der RPT-Vorrichtung (4000) bereitzustellen, und
wobei die RPT-Vorrichtung (4000) ferner ein zweites Gebläse aufweist.

2. System (7000) nach Anspruch 1, aufweisend einen HEPA-Filter (6006), wobei der HEPA-Filter (6006) dazu konfiguriert ist, einen Wirkungsgrad von mindestens 99 % für Partikel von 0,3 µm oder größer aufzuweisen.

3. System (7000) nach Anspruch 1 oder 2, wobei ein Teil einer den Luftauslass (6010) umgebenden Fläche der Vorrichtung nicht eben ist.

4. System (7000) nach einem der Ansprüche 1 bis 3, aufweisend einen oder mehrere Abstandshalterbereiche (6024), die aus einem den Luftauslass (6010) der Luftfilterungsvorrichtung (6000) umgebenden Flächenbereich hervorstehen.

5. System (7000) nach einem der Ansprüche 1 bis 4, aufweisend einen Luftqualitätssensor (6020, 6022).

6. System (7000) nach Anspruch 5, wobei der Luftqualitätssensor (6020, 6022) zum Messen des Partikelgehalts in der die Vorrichtung umgebenden Umgebungsluft konfiguriert ist.

7. System (7000) nach Anspruch 6, wobei die Vorrichtung einen weiteren Luftqualitätssensor (6022) stromabwärts der Filtereinrichtung aufweist.

8. System (7000) nach einem der Ansprüche 1 bis 7, aufweisend ein Datenkommunikationssystem.

9. System (7000) nach Anspruch 8, wobei das Datenkommunikationssystem ein drahtloses Datenkommunikationssystem ist.

10. System (7000) nach Anspruch 8 oder 9, wobei das Datenkommunikationssystem Daten übermittelt, die sich auf eines oder mehrere der folgenden beziehen: eine Durchflussrate durch die Vorrichtung; eine Partikelmenge und/oder VOCs in der Umgebungsluft; und eine Partikelmenge und/oder VOCs stromabwärts des HEPA-Filters (6006).

11. System (7000) nach einem der Ansprüche 1 bis 10, wobei der Bypass-Strömungsweg zum Ermöglichen einer ausreichenden Bypass-Strömung für die RPT-Vorrichtung (4000) konfiguriert ist, um einen Auslassluftstrom mit einem therapeutischen Druck und einer therapeutischen Durchflussrate bereitzustellen.

12. System (7000) nach einem der Ansprüche 1 bis 11, wobei die Luftfilterungsvorrichtung (6000) einen nicht ebenen Flächenbereich aufweist, der den Luftauslass (6010) der Luftfilterungsvorrichtung (6000) umgibt.

## Revendications

1. Système (7000) de fourniture d'air sous pression positive à un patient pour une thérapie respiratoire, le système (7000) comprenant un dispositif (4000) de thérapie par pression respiratoire (RPT) ayant une entrée et une forme prédéterminée autour de l'entrée, le système (7000) comprenant en outre un appareil de filtration d'air (6000) pour amener un flux d'air filtré à l'entrée du dispositif RPT (4000), l'appareil de filtration d'air (6000) comprenant un trajet de flux d'entrée (6026), un trajet de flux de sortie (6028), une sortie d'air (6010) et un premier ventilateur (6004) conçu pour recevoir l'air du trajet de flux d'entrée (6026) et pour amener l'air au trajet de flux de sortie (6028) à une pression supérieure à la pression ambiante, le trajet de flux d'entrée (6026) comprenant un moyen de filtration ayant un filtre HEPA (6006) et/ou un filtre COV, et le premier ventilateur (6004) étant conçu pour amener l'air sous pression au trajet de flux de sortie (6028) à un débit d'au moins 5 L/min,
dans lequel
une sortie d'air (6010) de l'appareil de filtration d'air (6000) est située par rapport à une entrée d'air du dispositif RPT (4000) de telle sorte que l'entrée d'air du dispositif RPT (4000) reçoive l'air filtré depuis la sortie d'air (6010) de l'appareil de filtration d'air (6000),
la forme d'un boîtier (6002) de l'appareil de filtration d'air (6000) est conçue pour définir un trajet de flux en dérivation entre une surface extérieure (6008) du boîtier (6002), entourant la sortie d'air (6010) de l'appareil de filtration d'air (6000), et l'entrée du dispositif RPT (4000), qui permet à l'air ambiant de circuler vers l'entrée du dispositif RPT (4000) si le débit de l'appareil de filtration d'air (6000) n'est pas suffisant pour fournir la totalité du flux requis par le dispositif RPT (4000), et
le dispositif RPT (4000) comprend en outre un deuxième ventilateur.

2. Système (7000) selon la revendication 1,
comprenant un filtre HEPA (6006), le filtre HEPA (6006) étant conçu pour avoir une efficacité de filtrer au moins 99 % des particules de 0,3 µm ou plus.

3. Système (7000) selon la revendication 1 ou 2,
dans lequel une partie de la surface de l'appareil, entourant la sortie d'air (6010), est non plane.

4. Système (7000) selon l'une des revendications 1 à 3,
comprenant un ou plusieurs éléments d'espacement (6024) qui font saillie depuis une partie de la surface entourant la sortie d'air (6010) de l'appareil de filtration d'air (6000).

5. Système (7000) selon l'une des revendications 1 à 4,
comprenant un capteur de qualité de l'air (6020, 6022).

6. Système (7000) selon la revendication 5,
dans lequel le capteur de qualité de l'air (6020, 6022) est conçu pour mesurer les niveaux de particules dans l'air ambiant entourant l'appareil.

7. Système (7000) selon la revendication 6,
dans lequel l'appareil comprend un autre capteur de qualité de l'air (6022) en aval du moyen de filtration.

8. Système (7000) selon l'une des revendications 1 à 7,
comprenant un système de communication de données.

9. Système (7000) selon la revendication 8,
dans lequel le système de communication de données est un système de communication de données sans fil.

10. Système (7000) selon la revendication 8 ou 9,
dans lequel le système de communication de données communique des données relatives à un ou plusieurs éléments parmi : le débit à travers l'appareil ; le niveau de particules et/ou de COV dans l'air ambiant ; et le niveau de particules et/ou de COV en aval du filtre HEPA (6006).

11. Système (7000) selon l'une des revendications 1 à 10,
dans lequel le trajet de flux en dérivation est conçu pour permettre un flux en dérivation suffisant pour que le dispositif RPT (4000) fournisse un flux d'air de sortie à une pression et un débit thérapeutiques.

12. Système (7000) selon l'une des revendications 1 à 11,
dans lequel l'appareil de filtration d'air (6000) comprend une partie de surface non plane entourant la sortie d'air (6010) de l'appareil de filtration d'air (6000).
